# EUROPÄISCHE PATENTSCHRIFT

(11) **EP 1 068 010 B1**
(45) Veröffentlichungstag und Bekanntmachung des Hinweises auf die Patenterteilung: **15.09.2004**
(21) Anmeldenummer: 99917905.4
(22) Anmeldetag: 29.03.1999
(51) Int. Cl.: B01J 23/78, B01J 23/83, C07C 5/333, C07C 15/46, B01J 35/10

(54) **KATALYSATOR ZUR DEHYDRIERUNG VON ETHYLBENZOL ZU STYROL**
CATALYST FOR DEHYDROGENATING ETHYL BENZENE TO PRODUCE STYRENE
CATALYSEUR POUR DESHYDROGENER DU BENZENE D'ETHYLE EN STYRENE

(30) Priorität: 30.03.1998 DE 19814080
(43) Veröffentlichungstag der Anmeldung: 17.01.2001
(73) Patentinhaber: BASF AKTIENGESELLSCHAFT, 67056 Ludwigshafen (DE)
(72) Erfinder: BAIER, Michael, D-68161 Mannheim (DE); HOFSTADT, Otto, D-67122 Altrip (DE); PÖPEL, Wolfgang, Jürgen, D-64297 Darmstadt (DE); PETERSEN, Hermann, D-67269 Grünstadt (DE)
(86) Internationale Anmeldenummer: PCT/EP1999/002146
(87) Internationale Veröffentlichungsnummer: WO 1999/049966

(56) Entgegenhaltungen:
- EP-A- 0 177 832
- EP-A- 0 794 004
- EP-A- 0 894 528
- EP-A- 1 071 508
- WO-A-96/18457
- WO-A-96/18594
- CA-A- 2 181 314
- DE-A- 2 815 812
- DE-A- 4 025 930

## Beschreibung

Die EP-A 0 181 999 beschreibt einen Dehydrierkatalysator der neben Fe₂O₃, K₂O und MgO zusätzlich Chrom- und/oder Mangan, eine Verbindung des Cer, Molybdäns oder Wolframs und CaO enthalten kann. Die in den Beispielen genannten Katalysatoren werden bei Temperaturen im Bereich von 510 bis 540°C calciniert. Es wird darauf hingewiesen, daß die Aktivität der Katalysatoren, insbesondere der selektiven Katalysatoren bei höheren Calciniertemperaturen erheblich herabgesetzt wird.

Eine niedere Calciniertemperatur (540°C) wird auch in EP-A 0 177 832 für magnesiumhaltige Katalysatoren auf Basis von Fe₂O₃ und K₂O beschrieben.

Die DE-A 28 15 812 beschreibt Dehydrierungskatalysatoren, die aus Gemischen von Eisenoxid, Kaliumoxid, Vanadiumoxid und gegebenenfalls Chromoxid bestehen. Durch geringe Mengen von Sauerstoff enthaltenden Verbindungen von Aluminium, Cadmium, Kupfer, Magnesium, Mangan, Nickel, Uran, Zink oder einer seltenen Erde und deren Gemische soll die Selektivität und/oder Umwandlungsrate zu ungesättigten Kohlenwasserstoffen aus gesättigten Verbindungen verbessert werden.

Die DE 38 21 431 beschreibt einen K₂Fe₂₂O₃₄ enthaltenden Katalysator, der bei 900°C calciniert wird. Zur Herstellung werden ausschließlich Eisenoxid und eine Kaliumverbindung eingesetzt. Das Calcinierprodukt wird anschließend gewaschen und filtriert, wobei das erhaltene Produkt lamellenförmige Plätten mit einem Durchmesser von 0,5 bis 5 µm sind.

Die CA-A-2 181 314 offenbart einen Dehydierkatalysator, der aus Gemischen von Eisenoxid, Kaliumoxid, Caliumoxid, Molybdänoxid, Ceroxid, Magnesiumoxid und Chromoxid besteht. Durch Reduzierung mit Wasserstoff soll die anfängliche Aktivität und die Lebenszeit verbessert werden.

Die EP-A-894 528 beschreibt die Herstellung eines Dehydrierkatalysators, in dem Kaliumferrat durch Reaktion von gelbem Eisenoxid (FeOOH) in einer wässrigen Lösung aus Cersalz mit wässrigem Kaliumhydroxid hergestellt wird. Das Kaliumferrat wird extrudiert, getrocknet und kalziniert bevor es mit weiteren Dotiermetallen, wie Calcium, Magnesium, Molybdän oder Wolfram, vermischt wird.

Die WO 99/49968 offenbart einen Dehydierkatalysator aus Eisenoxid und Alkalimetalloxiden. Die Selektivität und die Aktivität des Katalysators soll durch die Zugabe von geringen Mengen an Edelmetall, beispielsweise Rhodium, Ruthenium, Iridium, Rhenium und/oder Osmium gesteigert werden.

Aufgabe der Erfindung ist es, einen Katalysator mit verbesserterer Aktivität und Selektivität bei der Dehydrierung von Ethylbenzol zu Styrol bereitzustellen. Der Katalysator sollte außerdem eine hohe mechanische und chemische Stabilität und eine gute Lagerstabilität besitzen.

Demgemäß wurde ein Katalysator, enthaltend Eisenoxid, Kaliumoxid, eine Magnesiumverbindung und eine Cerverbindung, gefunden, wobei der Katalysator einen mittleren Porendurchmesser von über 0,35 µm aufweist und
50 - 90 Gew.-% Eisen, berechnet als Fe₂O₃,
1 - 40 Gew.-% Kalium, berechnet als K₂O,
5 - 20 Gew.-% Cer, berechnet als Ce₂O₃,
0,1 - 10 Gew.-% Magnesium, berechnet als MgO und
1 - 10 Gew.-% Calcium, berechnet als CaO, enthält,
mit der Maßgabe, dass zur Herstellung des Katalysators wenigstens 50 Gew.-% α-Fe₂O₃ bezogen auf die eingesetzten Eisenverbindungen mit einer mittleren längsten Teichenausdehnung von über 0,3 µm verwendet werden, der Katalysator eine oder mehrere Fe/K Phasen K₂O • Fe₂O₃ 1:n, mit einer natürlichen Zahl n von 1 bis 11, aufweist und der Katalysator bei einer Temperatur von über 750°C calciniert wird.

Zu den gegenüber den bekannten Katalysatoren besonderen Struktureigenschaften zählen große Porendurchmesser bei hoher mechanischer Stabilität, eine hohe innere Oberfläche, ein geringes Litergewicht und die deutliche Ausbildung von röntgenographisch erfaßbaren Fe/K-Phasen K₂O·Fe₂O₃ 1:4 und/oder K₂O·Fe₂O₃ 1:11. Die besten bisher bekannten, magnesiumhaltigen Katalysatoren enthalten aufgrund der niedrigeren Calciniertemperatur mit Ausnahme von geringen Mengen K₂Fe₂O₄ keine Fe/K-Phasen, sondern nur Fe₂O₃ (Hämatit) als Eisenbestandteil. Die Fe/K-Phasen K₂O·Fe₂O₃ 1:4 und K₂O·Fe₂O₃ 1:11 bilden sich offenbar erst ab 750°C.

Die Fe/K-Phasen können röntgenographisch bestimmt werden. Die Netzebenabstände und relativen Intensitäten der Fe/K-Phasen K₂O.Fe₂O₃ 1:5 (K₂Fe₁₀O₁₆) und/oder K₂O.Fe₂O₃ 1:11 (K₂Fe₂₂O₃₄) sind in Tabelle 3 zusammengefaßt. Aufgrund der Einlagerung von Magnesium, Cer und gegebenenfalls weiterer Promotor- und Zusatzmetallen in die Fe/K-Phasen, können die Reflexe geringfügig gegenüber den reinen Fe/K-Phasen verschoben sein.

Bevorzugte erfindungsgemäße Katalysatoren weisen Reflexe für Netzebenenabstände in folgenden Bereichen auf:

| | Netzebenenabstand d(Å) | +/- |
|---|---|---|
| 1. Reflex | 11,7 | 0,5 |
| 2. Reflex | 5,8 | 0,5 |
| 3. Reflex | 2,97 | 0,1 |
| 4. Reflex | 2,82 | 0,1 |
| 5. Reflex | 2,65 | 0,05 |
| 6. Reflex | 2,56 | 0,05 |
| 7. Reflex | 2,45 | 0,05 |
| 8. Reflex | 2,37 | 0,05 |
| 9. Reflex | 2,26 | 0,05 |
| 10. Reflex | 2,15 | 0,05 |
| 11. Reflex | 1,69 | 0,02 |
| 12. Reflex | 1,65 | 0,02 |
| 13. Reflex | 1,48 | 0,02 |

Besonders bevorzugte Katalysatoren weisen einen 1. Reflex im Bereich von 11,70 Å bis 11,90 Å, insbesondere 11,74 Å bis 11,87 Å und einen zweiten Reflex bei 5,85 Å bis 5,95 Å, insbesondere 5,89 Å bis 5,93 Å auf.

Weiterhin kann der Katalysator außer Magnesium und Cer ein oder mehrere weitere üblichen Promotoren zur Steigerung von Selektivität, Aktivität oder Stabilität in üblichen Konzentrationen enthalten. Als Promotoren eignen sich Verbindungen von Elementen ausgewählt aus der Gruppe Be, Ca, Sr, Ba, Sc, Ti, Zr, Hf, V, Ta, Mo, W, Mn, Tc, Re, Ru, Os, Co, Rh, Ir, Ni, Pd, Pt, Cu, Ag, Au, Zn, Cd, Hg, Al, Ga, In, Tl, Ge, Sn, Pb, Bi, Pr, Nd, Pm, Sm, Eu, Gd, Tb, Dy, Ho, Er, Tm, Yb und Lu, die einzeln oder in Mischungen verwendet werden können. Bevorzugte zusätzliche Promotoren sind Verbindungen ausgewählt aus der Gruppe Ca, V, Cr, Mo, W, Ti, Mn, Co und Al. Besonders bevorzugte zusätzliche Promotoren sind Ca, V, Cr, Mo und W. Die zusätzlichen Promotoren werden bevorzugt in Mengen von jeweils 0 bis 15, insbesondere 1 bis 10 Gew.-%, berechnet als stabilste Oxide, zugesetzt.

Kalium kann teilweise durch äquivalente Mengen anderer Alkalimetalle ersetzt werden z.B. Cäsium oder Natrium.

Bevorzugt enthält der erfindungsgemäße Katalysator Eisen, Kalium, Cer und Magnesium und als weitere Elemente Wolfram, Molybdän und Calcium. Günstig sind ferner Katalysatoren, die Vanadium enthalten.

Der Zusatz von Vanadium erhöht die Selektivität der Katalysatoren weiter. Deshalb ist der Zusatz von Vanadium (0,1-10 Gew.-%) sehr von Vorteil.

Elemente wie Cr, Al, Ti, Co, Li, Zn sind in den erfindungsgemäßen Katalysatoren in der Regel in untergeordneter Menge enthalten, z.B. 0 bis 2, insbesondere 0 bis 1 Gew.-%, jeweils als Oxid.

Bevorzugt enthalten die Katalysatoren jedoch kein Chrom.

Beispielsweise enthält ein erfindungsgemäßer Katalysator im gebrauchsfertigen Zustand:
50-90, insbesondere 60-80 Gew.-% Eisen, berechnet als Fe₂O₃;
1-40, insbesondere 5-15 Gew.-% Kalium, berechnet als K₂O;
5-20, insbesondere 6-15 Gew.-% Cer, berechnet als Ce₂O₃;
0,1-10, insbesondere 1- 5 Gew.-% Magnesium, berechnet als MgO;
0-10, insbesondere 0,1- 4 Gew.-% Calcium, berechnet als CaO;
0-10, insbesondere 0- 5 Gew.-% Wolfram, berechnet als WO₃;
0-10, insbesondere 0- 5 Gew.-% Molybdän, berechnet als MoO₃;
0-10, insbesondere 0,1- 4 Gew.-% Vanadium, berechnet als V₂O₅,
mit der Maßgabe, daß wenigstens 0,1 Gew.-%, insbesondere 1 Gew.-%, Wolfram oder Molybdän vorhanden sind.

Als Kaliumverbindung wird bevorzugt Kaliumcarbonat, Kaliumhydroxid oder eine andere in der Wärme zersetzbare Kaliumverbindung wie Kaliumoxalat verwendet. Man kann auch eine Kaliumverbindung verwenden, die den vorgesehenen Promotor (d.h. als entsprechendes Anion oder als Doppelsalz) enthält.

Als Vanadiumverbindung werden bevorzugt V₂O₅, Ammoniumvanadat oder Alkalimetallvanadate verwendet.

Als Magnesiumverbindung wird bevorzugt Mg(OH)₂, MgO, MgCO₃ oder Magnesiumhydroxycarbonat eingesetzt.

Als Cerverbindung wird bevorzugt Ce₂O₃, Ceroxalat oder Cercarbonat verwendet.

Als Molybdänverbindung wird bevorzugt MoO₃, H₂MoO₄ oder Ammoniummolybdat verwendet.

Als Wolframverbindung wird bevorzugt WO₃, H₂WO₄ oder Ammoniumwolframit verwendet.

Als Aluminiumverbindung wird bevorzugt AlOOH oder Al₂O₃ verwendet.

Als Calciumverbindung wird bevorzugt CaO, CaCO₃ oder Ca(OH)₂ verwendet.

Der erfindungsgemäße Katalysator wird überwiegend aus α-Fe₂O₃ anstelle des in EP-A-0195252 bevorzugt verwendeten FeOOH hergestellt und enthält eine gegenüber der dort angegebenen Empfehlung von 3-6 Gew.-% Ce₂O₃ erhöhte Cermenge von bis zu 20 %, berechnet als Ce₂O₃. Die in den neuen Katalysatoren verwendete erhöhte Cermenge führt zu einer Verbesserung der Aktivität und der Langzeitstabilität.

Katalysatoren, die unter Verwendung von α-Fe₂O₃ (Hämatit) mit einer mittleren längsten Teilchenausdehnung von über 0,3 µm erhalten wurden, weisen einen Porendurchmesser entsprechender Größe auf. Bevorzugt weisen die Katalysatoren einen mittleren Porendurchmesser von über 0,40 µm auf. Reines Eisenoxidhydroxid (FeOOH) eignet sich weniger für die Katalysatorherstellung. Untersuchungen an entsprechenden Vergleichskatalysatoren zeigen, daß diese weder die gewünschten großen Porendurchmesser noch eine befriedigende mechanische Stabilität aufweisen.

Statt reinem α-Fe₂O₃ (Hämatit) können jedoch Mischungen von α-Fe₂O₃ und α-FeOOH (Goethit) als Eisenkomponente eingesetzt werden, sofern, wie oben bereits aufgeführt, wenigstens 50 Gew.-% α-Fe₂O₃ bei der Herstellung verwendet werden. Bevorzugt sind Mischungen von 60 bis 90 Gew.-% α-Fe₂O₃ und 10 bis 40 Gew.-% α-FeOOH. Ein aus einer Mischung von z.B. 70 Gew.-% Fe₂O₃ und 30 Gew.-% FeOOH nach dem erfindungsgemäßen Verfahren hergestellter Katalysator hat zwar im Durchschnitt etwas kleinere Porenradien und eine geringfügig geringere mechanische Stabilität, dafür jedoch eine deutlich höhere innere Oberfläche als der nur aus Fe₂O₃ hergestellte Katalysator. Die Folge ist eine weitere Verbesserung der Aktivität.

Die Herstellung folgt im Prinzip dem in der EP A 0 195 252 Verfahren, jedoch wird bei deutlich höheren Temperaturen calciniert. Die Verbesserung der neuen Katalysatoren gegenüber diesen bekannten und den sonst beschriebenen Katalysatoren beruht offenbar darauf, daß neben der Verwendung einer bestimmten Eisenmodifikation, Magnesium und einer höheren Cermenge bei sonst vergleichbarem Verfahren bei Temperaturen von über 750°C, bevorzugt bei Temperaturen im Bereich von 760 bis 1000°C, insbesondere 800 bis 900°C, calciniert wird. Die höhere Calciniertemperatur führt zusammen mit der höheren Cermenge und dem Gehalt an Magnesium zu neuen ungewöhnlichen Eigenschaften der Katalysatoren und gleichzeitig zu einer deutlichen Verbesserung von Aktivität und Selektivität. Es wurde gefunden, daß dieser Katalysator schon bei niedrigen Dampf/EB-Verhältnissen (bis D/EB = 1,0 kg/kg) eine hervorragende Leistungsfähigkeit und Langzeitstabilität bei guter mechanischer Stabilität besitzt. Er hat also auch wegen des geringeren Energieverbrauchs des damit betriebenen Verfahrens wirtschaftliche Vorteile gegenüber den bekannten Katalysatoren. Aber auch bei den gegenwärtig üblichen höheren Dampf/EB-Verhältnissen von 1,1-1,5 kg/kg erreicht der neue Katalysator eine bessere Leistung.

Die Erfindung betrifft auch das Verfahren zur Dehydrierung alkylaromatischer oder aliphatischer Kohlenwasserstoffe zu entsprechenden Alkenen unter Verwendung der erfindungsgemäßen Katalysatoren.

Besonders bevorzugt ist die Dehydrierung von Ethylbenzol zu Styrol. Die Katalysatoren können aber vorteilhaft auch für die Dehydrierung von 1,1-Diphenylethan (DPEA) zu 1,1-Diphenylethen (DPE) eingesetzt werden. DPE ist als Rohstoff für Styrolcopolymere mit erhöhter Temperaturbeständigkeit gesucht.

Zur Herstellung der erfindungsgemäßen Katalysatoren ist im einzelnen das Folgende zu sagen:
Mischen der Einsatzstoffe und Herstellung einer verformbaren Masse

Wichtig ist eine innige Durchmischung der Einsatzstoffe. Diese kann einfach durch trockene Mischung oder durch Suspendierung der Einsatzstoffe in Wasser und Sprühtrocknung der gebildeten Maische hergestellt werden. Bei allen Mischvorgängen ist es von Vorteil alle Bestandteile mit Ausnahme des Eisenoxids in möglichst feingepulverter Form vorzulegen. Aus den Mischungen erhält man nach Wasserzugabe durch Kneten eine verformbare Masse. Die Verformung zu Tabletten kann dagegen mit einer trockenen Mischung der Bestandteile durchgeführt werden.

### Herstellung von Formkörpern

Formkörper lassen sich außer durch Extrusion oder Verstrangung auch durch Tablettierung des trockenen Sprühpulvers oder eines Gemisches in einer Tablettenpresse herstellen. In diesem Fall kann es von Nutzen sein, Tablettierungshilfsmittel (z.B. Graphit, verschiedene Stearate) zum Tablettiergut zuzusetzen. Als Formkörper kommen Stränge unterschiedlicher Geometrie in Frage, bevorzugt Vollstränge mit einem Durchmesser von z.B. 3-6 mm. Ebenso geeignet sind z.B. Hohlstränge oder sog. Rib- oder Sternstränge (d.h. Stränge mit äußeren Längsrillen oder rippen bzw. sternförmigem Querschnitt) oder Ringtabletten mit Innenloch. Die Verformbarkeit kann mit Hilfsmitteln wie Stearaten, Walocel, Stärke oder ähnlichem beeinflußt werden.

### Trocknung

Die Trocknung kann kontinuierlich oder diskontinuierlich durchgeführt werden. Für die kontinuierliche Trocknung kommen Bandtrockner, für die diskontinuierliche Trocknung Hordenöfen in Frage. Im technischen Maßstab sind die für großtechnische Trockenverfahren geeigneten Anlagen verwendbar wie Bandtrockner oder Hordenöfen. Übliche Trocknungstemperaturen sind 80-140°C. Höhere Trocknungstemperaturen können aufgrund einer zu hohen Trocknungsgeschwindigkeit zu einem unerwünschten Aufplatzen der Formkörper führen. Niedrigere Trocknungstemperaturen sind möglich, verlängern aber die Trocknungszeit.

### Temperung; Calcinierung

Nach dem Trocknen werden die Formkörper zuerst für ca. 2 h auf 250-350°C ("Temperung"), dann für 1-2 h auf 750-1000°C erhitzt ("Calcinierung"). Bei der Herstellung in technischem Maßstab kann das Tempern und Calcinieren in einem Arbeitsgang z.B. in einem Drehrohr mit unterschiedlichen Heizzonen durchgeführt werden. Die Temperatur wird dann schrittweise von z.B. 250 auf 800-900°C erhöht. Nach dem Austritt aus dem Drehrohr läßt man die Formkörper abkühlen. Bruchstücke und Feinstaub werden durch Siebung abgetrennt und verworfen.

### Herstellbeispiele; Vergleichsversuche

### (Zusammensetzung und Eigenschaften zusammengefaßt in den nachstehenden Tabellen).

Zur Herstellung des erfindungsgemäßen Katalysators gemäß Beispiel 1 wurde verfahren, wie nachstehend beschrieben. Die Katalysatoren der übrigen Beispiele wurden unter entsprechender Änderung der Mengenverhältnisse auf analoge Weise erhalten.

900 g α-Fe₂O₃, das in Form von Nadeln mit einer Länge von 0,4 µm und einem Verhältnis "Länge/Breite" von ca. 5 vorlag, wurden unter Rühren zu einer Suspension gegeben, die außerdem 200 g K₂CO₃, 200 g wasserhaltiges Cercarbonat (Zusammensetzung der Formel Ce₂(CO₃)₃·xH₂O mit einem Cergehalt von 40 Gew.-%), 40 g CaCO₃, 40 g WO₃ und 60 g basisches Magnesiumcarbonat (Zusammensetzung der Formel 4MgCO₃·Mg(OH)₂·4H₂O entsprechend 50 g MgCO₃) in 2000 ml Wasser enthielt. Die Suspension wurde sprühgetrocknet. Das Sprühpulver wurde in einem Kneter unter Zugabe von ca. 120 ml Wasser innerhalb von 30 Minuten zu einer pastosen Masse verarbeitet. Es war nicht notwendig, die Verformbarkeit mit Hilfsmitteln zu beeinflussen. Die Masse wurde in einer Strangpresse zu zylindrischen Vollsträngen von 3 mm Durchmesser geformt, in ca. 1 cm lange Stücke geschnitten, in einem Umluftofen bei 100°C ca. 3 Stunden getrocknet und in einem Calcinierofen erst 2 Stunden bei 300°C getempert und dann bei 760°C calciniert.

Bei Beispiel 8 und den Vergleichsversuchen V1 und V2 wurde Fe₂O₃ teilweise oder vollständig durch die entsprechende Menge α-FeOOH (nadelförmiges Eisenoxidgelb mit einer Nadellänge von ca. 0,6 µm und einem Längen/Durchmesser-Verhältnis von ca. 6) substituiert. Sobald das Eisenoxid zugegeben war, wurde weiteres starkes Rühren oder Kneten (starker Krafteintrag) vermieden, da er zu einer Zerkleinerung der Eisenoxidpartikel führen kann und damit die Eigenschaften des Katalysators verschlechtert.

**Tabelle 1**

| Zusammensetzung erfindungsgemäßer Katalysatoren in Gewichtsteilen (bei der Herstellung eingesetzte Mengen der Bestandteile in [dg]) | | | | | | | | | |
|---|---|---|---|---|---|---|---|---|---|
| Beispiel | 1 | 2 | 3 | 4 | 5 | 6 | 7 | 8 | 9 |
| Fe₂O₃ | 90 | 90 | 90 | 90 | 90 | 90 | 90 | 63 | 90 |
| FeOOH | - | - | - | - | - | - | - | 30 | |
| K₂CO₃ | 20 | 20 | 20 | 20 | 20 | 20 | 20 | 20 | 16, 6 |
| Ce₂(CO₃)₃·xH₂O | 20 | 20 | 20 | 20 | 10 | 20 | 20 | 20 | 20 |
| CaCO₃ | 4 | 4 | 4 | 4 | 4 | 4 | 4 | 4 | 4 , 6 |
| WO₃ | 4 | 4 | 4 | 4 | 4 | - | 4 | 4 | - |
| MoO₃ | - | - | - | - | - | 2,5 | - | - | 2,9 |
| 4MgCO₃·Mg(OH)₂·4H₂O | 5, 0 | 5,0 | 5, 0 | 5, 0 | 5, 0 | 5, 0 | 5, 0 | 5, 0 | 6,1 |
| V₂O₅ | - | - | - | - | - | - | 1,5 | - | - |
| T_{Calc} [°C] | 760 | 800 | 850 | 900 | 850 | 850 | 850 | 850 | 875 |

**Tabelle 2**

| Zusammensetzung der Vergleichsversuche V1 bis V4 (Mengenangaben wie vorstehend) | | | | |
|---|---|---|---|---|
| Vergleich | V1 | V2 | V3 | V4 |
| FeOOH | 100 | 100 | - | - |
| Fe₂O₃ | - | - | 90 | 90 |
| K₂CO₃ | 20 | 20 | 20 | 20 |
| Ce₂(CO₃)₃·xH₂O | 10 | 20 | 20 | 20 |
| CaCO₃ | 4 | 4 | 4 | 4 |
| WO₃ | 4 | 4 | 4 | 4 |
| 4MgCO₃·Mg(OH)₂·4H₂O | - | 5,0 | - | 5,0 |
| V₂O₅ | - | - | - | - |
| T_{Calc} [°C] | 850 | 850 | 850 | 700 |

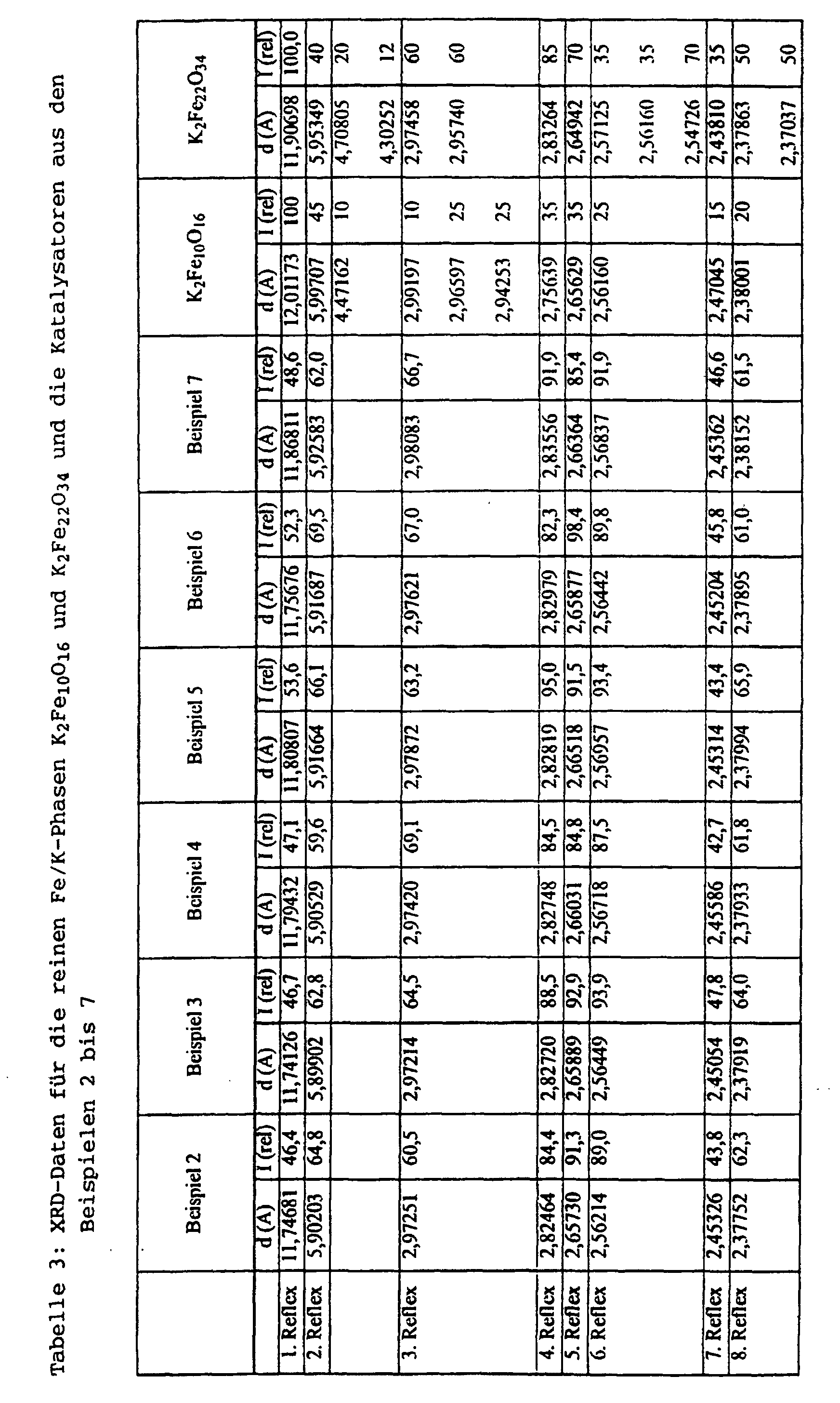

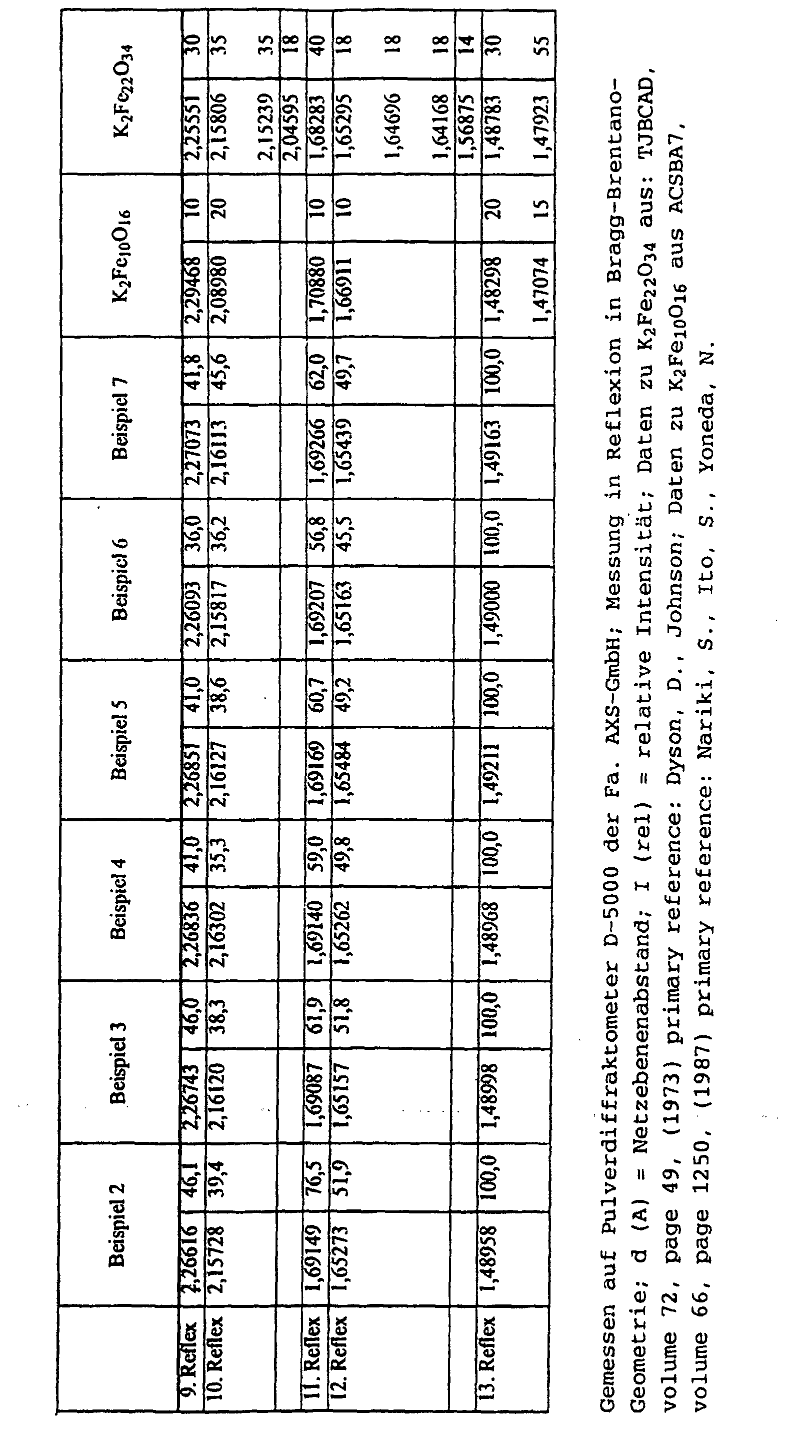

### Leistungstests

Die Katalysatoren werden in einer Versuchseinrichtung getestet, die dem technisch betriebenen isothermen Verfahren nachgebildet ist. Dazu werden 200 ml Vollstrang-Katalysator in ein Reaktionsrohr mit 30 mm Innendurchmesser gefüllt. Zunächst wird während 10 Tagen eine stündliche Menge von 183 ml Wasser und 168 ml Ethylbenzol in Dampfform übergeleitet. Die Katalysatortemperatur wird auf 600°C eingestellt. Nach 10 Tagen werden Umsatz (U), Selektivität (S) und Zusammensetzung des Reaktionsgemisches ermittelt, indem die Reaktionsprodukte (Flüssigkeit und Abgas) untersucht werden.

### Erläuterung der Tabellen:

Die erfindungsgemäßen Katalysatoren 1-8 liefern bei gleicher Temperatur durchweg höhere Umsätze als die Vergleichskatalysatoren V1 und V3. Damit hat der Einsatz dieser Katalysatoren an Stelle der Vergleichskatalysatoren den Vorteil, daß Energiekosten eingespart werden können (Reaktor, destillative Aufarbeitung). Vergleichskatalysator V2 erzielt bei gleicher Temperatur zwar vergleichbare Umsätze wie die erfindungsgemäßen Katalysatoren. Allerdings ist die Schnitthärte und damit auch die mechanische Stabilität zu gering, um ihn großtechnisch einsetzen zu können. Katalysator V4 hat dagegen sowohl bei der Aktivität (geringere Umsätze bei gleicher Temperatur) als auch bei der Selektivität Nachteile gegenüber den erfindungsgemäßen Katalysatoren.

**Tabelle 4**

| Eigenschaften und Leistung der erfindungsgemäßen Katalysatoren | | | | | | | | | |
|---|---|---|---|---|---|---|---|---|---|
| Beispiel | 1 | 2 | 3 | 4 | 5 | 6 | 7 | 8 | 9 |
| Schnitthärte [N] | 46 | 40 | 52 | 51 | 70 | 55 | 40 | 38 | 75 |
| BET-Oberfläche [m2/g] | 2,0 | 2,4 | 2,4 | 3,5 | 2,6 | 2,7 | 2,3 | 3,9 | 2,6 |
| Porenvolumen [ml/g] | 0,24 | 0,26 | 0,26 | 0,25 | 0,25 | 0,24 | 0,25 | 0,28 | 0,23 |
| mittlerer Poren-durchmesser [µm] | 0,46 | 0,45 | 0,47 | 0,46 | 0,43 | 0,45 | 0,42 | 0,32 | 0,38 |
| U_{600°C} | 50,6 | 50,8 | 50,8 | 49,3 | 47,3 | 50,3 | 48,6 | 52,3 | 50,8 |
| S_{600°C} | 95,4 | 96,0 | 96,4 | 96,5 | 96,6 | 96,5 | 97,0 | 96,2 | 96,4 |

**Tabelle 5**

| Eigenschaften und Leistung der Vergleichskatalysatoren | | | | |
|---|---|---|---|---|
| Vergleich | V1 | V2 | V3 | V4 |
| Schnitthärte [N] | 35 | 17 | 60 | 68 |
| BET-Oberfläche [m2/g] | 3,2 | 6,7 | 2,6 | 2,0 |
| Porenvolumen [ml/g] | 0,25 | 0,32 | 0,23 | 0,22 |
| mittlerer Porendurchmesser [µm] | 0,24 | 0,21 | 0,20 | 0,38 |
| U_{600°C} | 44,1 | 49,3 | 46,2 | 47,2 |
| S_{600°C} | 96,8 | 96,2 | 96,7 | 95,0 |

Die Porenvolumen wurden nach DIN-Norm 66133 bestimmt.

Der Kontaktwinkel des Quecksilbers bei der Bestimmung des mittleren Porendurchmessers betrug 140° (DIN 66133).

Zur Bestimmung der Schnitthärte wurde mit einer Schneide von 0,3 mm eine zunehmende Belastung auf das Extrudat ausgeübt, bis dieses zerschnitten war (Gerät der Fa. Zwick (Ulm)). Aus 25 Strängen wurde der Mittelwert gebildet.

## Patentansprüche

1. Katalysator, enthaltend Eisenoxid, Kaliumoxid, eine Magnesiumverbindung und eine Cerverbindung, wobei der Katalysator einen mittleren Porendurchmesser von über 0,35 µm aufweist und
50-90 Gew.-% Eisen, berechnet als Fe₂O₃,
1-40 Gew.-% Kalium, berechnet als K₂O,
5-20 Gew.-% Cer, berechnet als Ce₂O₃,
0,1-10 Gew.-% Magnesium, berechnet als MgO und
1-10 Gew.-% Calcium, berechnet als CaO,
enthält, mit der Maßgabe, dass zur Herstellung des Katalysators wenigstens 50 Gew.-% α-Fe₂O₃ bezogen auf die eingesetzten Eisenverbindungen mit einer mittleren längsten Teilchenausdehnung von über 0,3 µm verwendet werden, der Katalysator eine oder mehrere Fe/K-Phasen K₂O · Fe₂O₃ 1:n, mit einer natürlichen Zahl n von 1 bis 11, aufweist und der Katalysator bei einer Temperatur von über 750°C calciniert wird.

2. Katalysator nach Anspruch 1, **dadurch gekennzeichnet, daß** der Katalysator bei einer Temperatur im Bereich von 800 bis 900°C calciniert wurde.

3. Katalysator nach Anspruch 1 oder 2, **dadurch gekennzeichnet, daß** zur Herstellung des Katalysators als Eisenverbindung eine Mischung aus 60-90 Gew.-% α-Fe₂O₃ und 10-40 Gew.-% α-FeOOH verwendet wurde.

4. Verfahren zur Herstellung eines Katalysators gemäß den Ansprüchen 1 bis 3, **dadurch gekennzeichnet,daß** man aus α-Fe₂O₃ mit einer mittleren längsten Teilchenausdehnung von über 0,3 µm, einer Kaliumverbindung, einer Magnesiumverbindung, einer Cerverbindung und gegebenenfalls weiteren Katalysatorkomponenten einen Katalysatorformkörper herstellt und bei Temperaturen über 750°C calciniert.

5. Verfahren zur Dehydrierung von Ethylbenzol zu Styrol, **dadurch gekennzeichnet, daß** man die Dehydrierung in Gegenwart eines Katalysators nach einem der Ansprüche 1 bis 3 durchführt.

## Claims

1. A catalyst comprising iron oxide, potassium oxide, a magnesium compound and a cerium compound, in which the catalyst has a mean pore diameter of greater than 0.35 µm, and comprises
50-90% by weight of iron, calculated as Fe₂O₃,
1-40% by weight of potassium, calculated as K₂O,
5-20% by weight of cerium, calculated as Ce₂O₃,
0.1-10% by weight of magnesium, calculated as MgO and
1-10% by weight of calcium, calculated as CaO, with the proviso that the catalyst is prepared using at least 50% by weight of α-Fe₂O₃, based on the iron compounds employed, having a mean longest particle dimension of greater than 0.3 µm, the catalyst has one or more Fe/K phases K₂O·Fe₂O₃ 1:n, where n is a natural number from 1 to 11, and the catalyst is calcined at a temperature of above 750°C.

2. A catalyst as claimed in claim 1, wherein the catalyst has been calcined at a temperature in the range from 800 to 900°C.

3. A catalyst as claimed in claim 1 or 2, wherein the catalyst has been prepared using a mixture of 60-90% by weight of α-Fe₂O₃ and 10-40% of α-FeOOH as iron compound.

4. A process for the preparation of a catalyst as claimed in claims 1 to 3, wherein a catalyst molding is prepared from α-Fe₂O₃ having a mean longest particle dimension of greater than 0.3 µm, a potassium compound, a magnesium compound, a cerium compound and, if desired, further catalyst components, and is calcined at temperatures above 750°C.

5. A process for the dehydrogenation of ethylbenzene to styrene, wherein the dehydrogenation is carried out in the presence of a catalyst as claimed in one of claims 1 to 3.

## Revendications

1. Catalyseur contenant de l'oxyde de fer, de l'oxyde de potassium, un composé du magnésium et un composé du cérium, le catalyseur présentant un diamètre moyen des pores supérieur à 0,35 µm et contenant
50-90 % en poids de fer, calculé en tant que Fe₂O₃,
1-40 % en poids de potassium, calculé en tant que K₂O,
5-20 % en poids de cérium, calculé en tant que Ce₂O₃,
0,1-10 % en poids de magnésium, calculé en tant que MgO et
1-10 % en poids de calcium, calculé en tant que CaO,
sous réserve que pour la préparation du catalyseur, on utilise au moins 50 % en poids, par rapport aux composés du fer mis en oeuvre, de α-Fe₂O₃ ayant une dimension maximale moyenne des particules supérieure à 0,3 µm, le catalyseur présente une ou plusieurs phases Fe/K telles que K₂O · Fe₂O₃ 1 : n, n étant un nombre naturel de 1 à 11, et le catalyseur est calciné à une température supérieure à 750°C.

2. Catalyseur selon la revendication 1, **caractérisé en ce que** le catalyseur est calciné à une température comprise dans la gamme de 800 à 900°C.

3. Catalyseur selon la revendication 1 ou 2, **caractérisé en ce que** pour la préparation du catalyseur on utilise, en tant que composé du fer, un mélange constitué par 60-90 % en poids de α-Fe₂O₃ et 10-40 % en poids de α-FeOOH.

4. Procédé de préparation d'un catalyseur selon l'une quelconque des revendications 1 à 3, **caractérisé en ce que** l'on prépare, à partir de α-Fe₂O₃ ayant une dimension maximale moyenne des particules supérieure à 0,3 µm, d'un composé du potassium, d'un composé du magnésium, d'un composé du cérium et éventuellement d'autres composants de catalyseur, un corps moulé de catalyseur que l'on calcine à une température supérieure à 750°C.

5. Procédé de déshydrogénation d'éthylbenzène en styrène, **caractérisé en ce que** l'on effectue la déshydrogénation en présence d'un catalyseur selon l'une quelconque des revendications 1 à 3.
